# EUROPÄISCHE PATENTSCHRIFT

(11) **EP 2 159 241 B1**
(45) Veröffentlichungstag und Bekanntmachung des Hinweises auf die Patenterteilung: **25.05.2011**
(21) Anmeldenummer: 09157761.9
(22) Anmeldetag: 09.04.2009
(51) Int. Cl.: C08G 18/79, C08G 18/09, C07D 229/00

(54) **Verfahren zur Herstellung von oligomeren Diphenylmethan-4,4'- und/oder Diphenylmethan-2,4'-diisocyanat-uretdionen**
Method for producing oligomeric diphenylmethane-4,4' and/or diphenylmethan-2,4'-diisocyanate-uretdione
Procédé de fabrication de diphénylméthane-4,4'- et/ou de diphénylméthane-2,4'-diisocyanates-uretdiones oligomères

(43) Veröffentlichungstag der Anmeldung: 03.03.2010
(73) Patentinhaber: Isochem Kautschuk GmbH, 60313 Frankfurt (DE)
(72) Erfinder: Abend, Thomas, 6605, Locarno-Monti (CH); Schröter, Axel, 60313, Frankfurt (DE)
(74) Vertreter: Hepp, Dieter

(56) Entgegenhaltungen:
- EP-A- 0 071 899
- EP-A- 0 418 639
- GB-A- 1 088 580
- US-A- 3 290 288
- US-A- 4 888 124

## Beschreibung

Die vorliegende Erfindung betrifft ein verbessertes Verfahren zur Herstellung von oligomeren Diphenylmethan-4,4'-diisocyanat-uretdionen und/oder Diphenylmethan-2,4'-diisocyanat-uretdionen.

Bei der Herstellung von faserverstärkten Kautschukprodukten werden Haftvermittler zur Verbesserung der Haftfestigkeit zwischen textiler Verstärkungseinlage und dem Kautschuk eingesetzt. Unerlässlich ist der Einsatz eines Haftvermittlers im Bereich der hochbelasteten Verbundwerkstoffe mit Verstärkungsfasern, wie in Reifen, Antriebsriemen und Transportbändern. Als Verstärkungsmittel werden hierbei Fasern aus Polyamid, Aramid, Polyester, Rayon, usw. verwendet.

Nach dem Stand der Technik werden als Haftvermittlersysteme für solche Anwendungen überwiegend Resorcin-Formaldehyd-Latexsysteme verwendet, welche mit blockierten Polyisocyanaten und Epoxyharzen vernetzt sind. Polyisocyanate und Epoxyharze dienen durch ihre Reaktion mit den reaktionsfähigen Gruppen der Fasermoleküle auch als Aktivierungsmittel der Oberfläche der Fasermaterialien.

Die Haftvermittlersysteme werden für diese Anwendungen aus verdünnter wässeriger Dispersion auf die Faser aufgebracht, wobei zwei Verfahren unterschieden werden: Zweischrittverfahren und Einschrittverfahren.

Im älteren Zweischrittverfahren für die Ausrüstung von nicht voraktivierten Fasern, speziell von Polyesterfasern, wird zuerst
a) eine verdünnte wässerige Dispersion von blockierten Isocyanaten und wasserlöslichen Epoxyharzen durch einen Tauch- und Abquetschprozess auf die Faser oder auf ein textiles Flächengebilde aufgebracht, im Umluftofen getrocknet und bei 220 - 240 °C während ungefähr 60 Sekunden vernetzt, und
b) anschliessend eine verdünnte wässrige Dispersion aus Resorcin-Formaldehydharz und Styrol-Butadien-Vinylpyridin-Polymer durch einen Tauch- und Abquetschprozess auf die aktivierte Faser aufgebracht und, nach Trocknung bei 110 bis 140 °C während 120 bis 180 Sekunden im Umluftofen, bei 220 bis 240 °C Lufttemperatur während etwa 90 Sekunden vernetzt.

Im Einschrittverfahren, welches auf voraktivierten Fasermaterialien angewendet werden kann und verfahrenstechnisch weniger aufwändig ist, wird eine verdünnte wässerige Dispersion, enthaltend
blockiertes Polyisocyanat
wasserlösliches oder wasserverdünnbares Epoxydharz
Resorcin-Formaldehydharz-Lösung
Dispersion von Styrol-Butadien-Vinylpyridin-Polymer durch einen Tauch- und Abquetschprozess aufgebracht, im Ofen bei einer Lufttemperatur von 110 °C bis 150 °C während 120 Sekunden getrocknet und anschliessend bei 220 bis 240 °C während etwa 90 Sekunden vernetzt. Im Einschrittverfahren können auch nicht voraktivierte Fasermaterialien behandelt werden.

Fasern oder textile Flächengebilde aus den genannten Materialien, die gemäss dem vorstehend beschriebenen Ein- oder Zweischrittverfahren behandelt sind, zeigen ausgezeichnete Haftung mit vernetzten Kautschuken und Elastomeren.

Die vorstehenden Aspekte sind unter anderem in der EP 1 221 456 A1 oder der WO 2007/051562 beschrieben.

Für die vorgenannten Verfahren werden blockierte Polyisocyanate, vor allem auf Diphenylmethan-diisocyanat-Basis (MDI) verwendet, wobei die Isocyanatgruppen durch Blockierung vor der Reaktion mit Wasser geschützt werden müssen. Als Blockierungsmittel für die NCO-Gruppen werden vor allem ε-Caprolactam, Phenole, Oxime, Enol-bildende Verbindungen, sekundäre aliphatische Amine oder Triazole eingesetzt.

Die Blockierungsmittel stellen einen grossen Anteil (40 bis 50 %) des Formelgewichtes des blockierten Diphenylmethandiisocyanates dar. Im Falle des Caprolactam-blockierten MDI beträgt der Gewichtsanteil des Blockierungsmittels 47 %, beim Phenol-blockierten MDI 43 %. Die Verwendung von Blockierungsmitteln für MDI bedeutet
a) mehr Materialaufwand
b) mehr Transport- und Lageraufwand
c) Belastung der Umluft und Abluft in den Öfen

Im Temperaturbereich von 150 bis 170 °C spalten die heute für das Einstufen- und Zweistufenverfahren verwendeten blockierten Isocyanate das Blockierungsmittel ab, und die freigesetzten Blockierungsmittel verteilen sich bei den erhöhten Temperaturen zwischen der Ofenluft und Abluft sowie den Faserpolymeren.

Als Alternative zum blockierten MDI wurde schon vorgeschlagen, MDI-Uretdione oder dimeres MDI (CAS Nr. 17589-24-1 für 4,4'-MDI-Uretdion resp. CAS Nr. 94158-57-3 für 2,4'-MDI-Uretdion) zu verwenden. Durch die Dimerisierung sind die Isocyanatgruppen im Uretdionring vor Reaktionen mit Wasser geschützt.

Dimeres 4,4'-MDI-Uretdion hat die folgende Strukturformel:

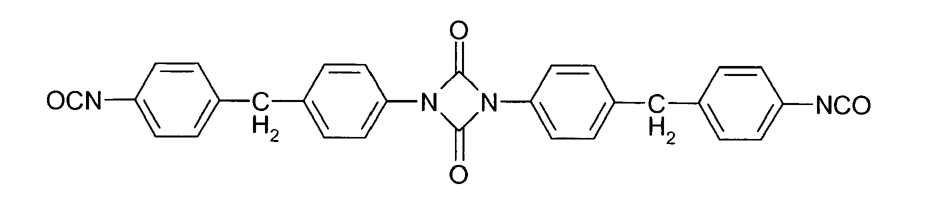

Dimeres 2,4'-MDI-Uretdion hat folgende Formel:

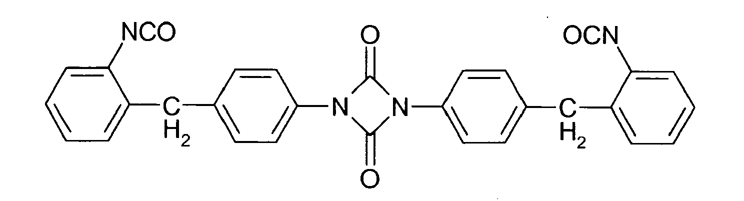

Das 4,4'-MDI, auch im Gemisch mit 2,4'-MDI, kann dimere, oligomere und niedermolekulare polymere Uretdione bilden. Das 2,4'-MDI allein bildet nur Dimere.

Die exotherme Dimerisierungs-/Oligomerisierungsreaktion wird in Lösung oder in Substanz durchgeführt. Zur Dimerisierung/Oligomerisierung von MDI werden Katalysatoren verwendet. Als Katalysatoren für die Dimerisierung/Oligomerisierung eignen sich u. a. Trialkylphosphine, speziell das Tributyl- oder Trioctylphosphin, Tris-(dialkyamino)-phosphine, substituierte Pyridine, speziell das 4-Dimethylamino-pyridin, substituierte Imidazole, speziell das 1,2-Dimethyl-imidazol, tertiäre Amine, heterocyclische Amine, wie N-Methlymorpholin, Amidine, etc.; es werden, bezogen auf die Menge des MDI, 0.01 bis 1 %, bevorzugt 0.1 bis 0.5 % Dimerisierungskatalysator eingesetzt.

Für die lösungsmittelfreie Herstellung in Substanz können beheizbare und gekühlte Reaktionsextruder verwendet werden, wie beispielsweise in der DE 24 52 390 beschrieben. Das resultierende Uretdion ist ein Pulver mit einer mittleren Korngrösse von 150 µm, welches für die meisten Anwendungen noch gemahlen werden muss. Auf S. 11, Z. 4 des DE 24 52 390 wird auch die Verwendung von MDI-Uretdionen als Haftvermittler für Reifencord vorgeschlagen.

Bei der Herstellung des Uretdions aus Lösung erhält man je nach MDI-Isomerenverhältnis, Lösungsmittel, der Art des Katalysators und der Reaktionstemperatur dimere, oligomere oder niedermolekulare, polymere MDI-Uretdione. Verwendet werden aprotische Lösungsmittel, wie aliphatische und aromatische Kohlenwasserstoffe, chlorierte Kohlenwasserstoffe, Ketone, Ether oder Ester.

Zur Herstellung des Dimeren oder Oligomeren wird MDI im Lösungsmittel gelöst oder in Form einer Schmelze bei 40 bis 80 °C zum Lösungsmittel gegeben.

Anschliessend wird der Katalysator zugegeben und im Temperaturbereich von Raumtemperatur bis 100 °C beziehungsweise dem Siedepunkt des Lösungsmittels, während mehreren Stunden dimerisiert. Je nach Lösungsparameter fällt mehr oder weniger Dimeres oder Oligomeres aus der Lösung aus und bildet eine feine Suspension. Am Ende der Reaktion kann der Katalysator neutralisiert oder zerstört und die Temperatur zur besseren Ausfällung des festen Uretdions erniedrigt werden.

Die Di- oder Oligomeren werden anschliessend filtriert oder zentrifugiert, mit Lösungsmittel gewaschen und (ggf. unter Vakuum) getrocknet.

Durch Erhitzen des Pulvers auf Temperaturen im Bereich von 80 - 100 °C während einigen Minuten bis Stunden kann ein Teil der endständigen Isocyanatgruppen weiter oligomerisiert und so der Gehalt an Rest-MDI-Monomer reduziert und der Polymerisationsgrad erhöht werden.

Der Herstellungsprozess und die Verwendung der dimeren oder oligomeren Pulver wurden unter anderem in der EP 071 898 A1, EP 195 917 A1, EP 418 639 A1 und der US 3,524,834 beschrieben.

Endprodukte der Synthese sind pulverförmige di-, oligo- oder polymere MDI-Uretdione mit einem Schmelzpunkt von 250 bis 260 °C für das 4,4'-MDI-Uretdion resp. 185 - 195 °C für das 2,4'-MDI-Uretdion, welche in unpolaren und polaren Lösungsmitteln schwer löslich sind. Das Dimer enthält theoretisch 16.8 % freies NCO. Die endständigen Isocyanatgruppen können mit Feuchtigkeit unter Harnstoffbildung reagieren. Beim oligomeren oder polymeren MDI-uretdion kann infolge deren Schwerlöslichkeit nur der totale NCO-Gehalt bestimmt werden, welches (nach Reversion des Uretdionringes zum Monomer) bis zu 30 % betragen kann. Die NCO-Bestimmung kann üblicherweise mit Dibutylamin in siedendem Dimethylformamid erfolgen.

Um die oligomeren MDI-Uretdione als blockierungsmittelfreie MDI-Spender in wässrige Haftvermittlerdispersionen überzuführen, sind nach dem Stand der Technik mehrere Verfahrenschritte notwendig:
a) Das MDI muss in einem Lösungsmittel unter Einwirkung von Katalysatoren oligomerisiert werden
b) Das entstehende Pulver muss aus der Lösung abgeschieden, mit Lösungsmittel gewaschen und getrocknet werden; eine Mahlung in einer Luftstrahlmühle kann sich anschliessen
c) Das Pulver wird im Normalfall in einer wässrigen Lösung, die auch Zusatzmittel enthält, dispergiert und
d) nass (beispielsweise in einer Perlmühle) auf mittlere Partikelgrössen von < 5 µm gemahlen.

Verluste an MDI-Uretdion und Lösungsmittel sind hierbei nicht zu vermeiden.

Es war die Aufgabe der vorliegenden Erfindung, ein verbessertes Verfahren bereitzustellen, mit welchem MDI-Uretdion auf einfache Weise ohne grössere, vorzugsweise ohne relevante Ausbeuteverluste bereitgestellt werden kann.

Diese Aufgabe wurde gemäss der vorliegenden Erfindung überraschend gelöst durch ein Verfahren zur Herstellung einer Dispersion oder Suspension von oligomeren Diphenylmethan-4,4'-diisocyanat-uretdionen und/oder Diphenylmethan-2,4'-diisocyanat-uretdionen, umfassend die aufeinander folgenden Schritte
i) Bereitstellung einer Reaktionsmischung enthaltend Diphenylmethan-4,4'-diisocyanat und/oder Diphenylmethan-2,4'-diisocyanat; und einen Dimerisierungs-Katalysator in einem aprotischen polaren Lösungsmittel oder einem Gemisch aprotischer polarer Lösungsmittel,
ii) Durchführung der Reaktion bei 20°C bis 100°C während 1 bis 8 Stunden
iii) gegebenenfalls Neutralisation oder Zerstörung des Katalysators
wobei
i) das polare aprotische Lösungsmittel wasserlöslich oder wassermischbar ist und unter Normalbedingungen einen Siedepunkt von 40°C bis 100°C aufweist oder mit Wasser ein unter 100°C siedendes Azeotrop bildet, welches nach Reaktionsende zumindest so weit durch Wasser ersetzt wird, dass eine fliessfähige wässrige Suspension oder Dispersion erhalten wird, die keinen Flammpunkt mehr aufweist; oder
ii) das polare aprotische Lösungsmittel wasserlöslich oder wassermischbar ist und unter Normalbedingungen einen Siedepunkt von 100°C bis 250°C aufweist.

Es wurde überraschend ein Weg gefunden, der die Isolierung des erhaltenen MDI-Uretdion-Pulvers nicht notwendig macht. Das Verfahren vermeidet einige Verfahrensschritte und ist praktisch verlustfrei.

Die Reaktion von Diphenylmethan-4,4'-diisocyanat und/oder Diphenylmethan-2,4'-diisocyanat liefert Dimere, Oligomere (3-5 Monomereinheiten) und niedermolekulare Polymeren (>5 Monomereinheiten). Sofern im Rahmen der vorliegenden Erfindung auf die Reaktion der genannten Diisocyanate Bezug genommen wird, so ist hierunter jeweils die Bildung von Dimeren, Oligomeren und niedermolekularen Polymeren zu verstehen, auch wenn im Einzelfall aus Gründen der Übersichtlichkeit nur von Oligomeren oder Dimeren gesprochen wird.

Gemäss der vorliegenden Erfindung wird eine Dispersion oder Suspension von oligomeren Diphenylmethan-4,4'-diisocyanaturetdionen erhalten. Es kann sich hierbei entweder um eine rein wässrige Dispersion oder Suspension oder um eine Suspension in einem polaren aprotischen Lösungsmittel und Wasser handeln.

Im Rahmen der vorliegenden Erfindung kann zusätzlich oder alternativ zu Diphenylmethan-4,4'-diisocyanat auch Diphenylmethan-2,4'-diisocyanat eingesetzt werden. Das reine Diphenylmethan-2,4'-diisocyanat ist recht teuer und liefert nur Dimere (was im Einzelfall gewünscht sein kann). Besonders bevorzugt sind Mischungen von Diphenylmethan-2,4'-diisocyanat und Diphenylmethan-4,4'-diisocyanat, da derartige Mischungen bei Normalbedingungen oder im Temperaturbereich von 23 °C bis 40 °C flüssig sind. Solche Mischungen werden von den Isocyanatherstellern BayerMaterialScience, BASF, Dow Chemical, Huntsman in den Handel gebracht. Sofern im Weiteren die Abkürzung MDI verwendet wird, sind stets all diese Ausführungsformen umfasst.

Um eine erfindungsgemässe wässrige Dispersion oder Suspension zu erhalten, wird die Reaktion zunächst in einem aprotischen polaren Lösungsmittel durchgeführt, wobei das polare aprotische Lösungsmittel wasserlöslich oder wassermischbar ist und unter Normalbedingungen einen Siedepunkt von 40°C bis 100°C aufweist oder unter Normalbedingungen mit Wasser ein unter 100°C siedendes Azeotrop bildet. Dadurch wird die Entfernung des Lösungsmittels ohne zu hohe Temperaturbelastung des Produkts ermöglicht.

Unter einem polaren Lösungsmittel oder Lösungsmittelgemisch wird ein solches verstanden, welches einen Löslichkeitsparameter nach Hansen (C. Hansen, A. Beerbower, in: Kirk-Othmer Encyclopedia of Chemical Technology, 2nd Edition, Suppl. Vol., New York, 1971, p. 889) von gleich oder mehr als 18.5 MPa^{½} beziehungsweise 9 cal^{½}cm^{3/2} aufweist.

Das Lösungsmittel soll zudem wasserlöslich oder wassermischbar sein. Gemäss der vorliegenden Erfindung wird unter "wassermischbar oder wasserlöslich" verstanden, dass bei Raumtemperatur mindestens 20 Gew.-%, bevorzugt über 30 Gew.-% Lösungsmittel in der Wasserphase löslich oder mit dieser mischbar sein sollen.

Nach Ende der Reaktion (und gegebenenfalls nach Zerstörung oder Neutralisation des Katalysators) wird die erhaltene Mischung mit einer definierten Menge Wasser oder einer Lösung eines primären oder sekundären Amins oder Polyamins in Wasser versetzt, um freie Isocyanate abzureagieren. Es wird hierbei eine solche Menge an Wasser und/oder Amin eingesetzt, dass (nach Abdestillieren des polaren aprotischen Lösungsmittels) eine fliessfähige wässrige Suspension oder Dispersion entsteht.

Alternativ kann die MDI-Uretdion-Suspension im Lösungsmittel auch in vorgelegtem Wasser bei Raumtemperatur oder erhöhter Temperatur (vorzugsweise etwa 30-50°C) dispergiert werden, ggf. unter Beigabe eines Dispergators, Emulgators oder weiterer Hilfsmittel.

Nach der Versetzung mit der definierten Menge an Wasser wird das polare aprotische Lösungsmittel weitgehend oder vollständig entfernt, bevorzugt bis die flüssige Phase der Dispersion keinen Flammpunkt mehr aufweist.

Es bieten sich für die Entfernung des polaren aprotischen Lösungsmittels aus der wässerigen Suspension oder Dispersion unter anderem folgende Methoden an:
- Das Lösungsmittel wird bei Normaldruck oder unter vermindertem Druck und/oder bei erhöhten Temperaturen abdestilliert, zum Beispiel in einem Dünnschicht- oder Kurzwegverdampfer.
- Das Gemisch wird gegebenenfalls unter vermindertem Druck versprüht und das Lösungsmittel hierbei weitgehend oder ganz entfernt
- Durch ein- oder mehrmaliges Zentrifugieren, Verwerfen der flüssigen Phase und Aufschlämmen des MDI-Uretdionpulvers mit Wasser wird der Gehalt an Lösungsmittel reduziert.

Unter Normalbedingungen (bzw. Normaldruck oder Normaltemperatur) werden im Rahmen der vorliegenden Erfindung 23°C und 1013,25 hPa verstanden.

Typischerweise wird die Suspension oder Dispersion auf einen Wassergehalt von > 50 Gew.%, bevorzugt > 70 Gew.%, besonders bevorzugt > 90 Gew.%, bezogen auf die flüssige Phase, eingestellt.

Beispiele von für diese erfindungsgemässe Variante geeigneten aprotischen, wasserlöslichen oder wassermischbaren Lösungsmitteln sind:

| Lösungsmittel | Sdp. °C; Lösungsmittel | Sdp. °C; Azeotrop mit Wasser | g Wasser in 100 g Azeotrop |
|---|---|---|---|
| Aceton | 56 | --- | --- |
| Methylethylketon | 79 | 73 | 12 |
| Tetrahydrofuran | 60 | 65 | 5 |
| 2-Methyltetrahydrofuran | 80 | 71 | 10 |
| 1,2-Dimethoxyethan | 85 | 78 | 10 |
| Dioxan | 101 | 88 | 18 |
| Methylacetat | 57 | 56 | 5 |

Da einige Dimerisierungskatalysatoren stark basisch reagieren und in der Dispersion verbleiben können, werden Ether und Ketone für diese Anwendung bevorzugt.

Gemäss der vorliegenden Erfindung können auch Gemische der vorstehenden Lösungsmittel eingesetzt werden.

Einige Lösungsmittel können azeotrope Mischungen mit Wasser bilden. Der geänderte Siedepunkt des Azeotrops sowie die entsprechende Wasserzugabe zum Lösungsmittel vor, beim oder nach dem Abdestillieren des Lösungsmittels, muss bei der Synthese oder beim Lösungsmittelaustausch berücksichtigt werden.

In einer alternativen Ausführungsform kann eine erfindungsgemässe Suspension oder Dispersion in einem polaren aprotischen Lösungsmittel und Wasser erhalten werden, wobei die Reaktion zunächst in einem aprotischen polaren Lösungsmittel durchgeführt wird, das polare aprotische Lösungsmittel wasserlöslich oder wassermischbar ist und unter Normalbedingungen einen Siedepunkt oder Siedebereich von 100 °C bis 250 °C, bevorzugt von 125 bis 220 °C aufweist.

Gemäss der vorliegenden Erfindung können auch Gemische der vorstehenden Lösungsmittel eingesetzt werden.

Das Lösungsmittel oder -gemisch kann mit Wasser ein Azeotrop bilden, welches im Bereich 90 bis 120 °C siedet, wobei Wasser im Azeotrop im Überschuss ist.

Als derartige erfindungsgemässe polare aprotische Lösungsmittel für diese Ausführungsform sind beispielsweise zu nennen:

| Lösungsmittel | Siedepunkt bei 1013 hPa, °C | Flammpunkt 100 % Lösungs-mittel |
|---|---|---|
| Diethylenglykol-diethylether | 188 | 82 |
| Diethylenglykol-dimethylether | 162 | 51 |
| Propylenglykoldimethylether | 180 | |
| Dipropylenglykol-dimethylether | 175 | 65 |
| N-Methyl-pyrrolidon | 204 | 95 |
| N-Ethyl-2-pyrrolidon | 212 | 91 |
| Cyclohexanon | 155 | 43 |
| Ethylen-/Propylencarbonat, 50 % Gemisch | 244 | 154 |

| Lösungsmittel | g löslich in 100 g wässriger Phase | Siedepunkt Azeotrop °C | g Lösungsmittel in 100 g Azeotrop |
|---|---|---|---|
| Diethylenglykol-diethylether | 100 | | |
| Diethylenglykol-dimethylether | 100 | 99 | 23 |
| Propylenglykol-dimethylether | 100 | | |
| Dipropylenglykol-dimethylether | 35 | 98 | 38 |
| N-Methyl-pyrrolidon | 100 | | |
| N-Ethyl-pyrrolidon | 100 | | |
| Ethylen-/Propylencarbonat, 50 % Gemisch | 48 | | |

Das polare Lösungsmittel kann mit dem Wasser ein Azeotrop bilden, welches sich bei der Trocknung bei Raumtemperatur oder bei erhöhten Temperaturen im Bereich 30 °C bis 250 °C verflüchtigt, insbesondere bei Applikationstemperatur auf Reifencord (typischerweise im Bereich von 100°C bis 220°C). Durch den Wasserüberschuss zeigen solche verdünnte Mischungen auch keinen Flammpunkt unter 65 °C.

Die gemäss dieser Ausführungsform erhaltene Suspension wird mit einer definierten Menge Wasser oder mit einer Lösung von primären oder sekundären Aminen oder Polyaminen und ggf. weiteren Hilfsmitteln oder Zusatzstoffen in Wasser versetzt. Alternativ kann die MDI-Uretdion-Suspension im Lösungsmittel auch in vorgelegtem Wasser bei Raum- oder erhöhter Temperatur dispergiert werden, gegebenenfalls unter Beigabe eines Dispergators, Emulgators oder weiterer Hilfsmittel. Derartige Dispergatoren, Emulgatoren oder Hilfsstoffe sind dem Fachmann bekannt.

Eine allfällige Oberflächen-Desaktivierung kann durchgeführt werden gemäss den aus EP 062780 und/oder EP 204970 (BASF-Coatings) bekannten Verfahren; die Offenbarung der vorgenannten Dokumente wird hinsichtlich der Oberflächen-Desaktivierung in die vorliegende Anmeldung durch Bezugnahme eingeschlossen.

Die Verwendung von polaren Lösungsmitteln oder ihren Gemischen für die Oligomerisierung bei erhöhten Temperaturen beziehungsweise Siedetemperatur des Lösungsmittels hat auch die folgenden erwünschten Wirkungen im Vergleich zur Verwendung von unpolaren Lösungsmitteln:
a) Monomeres 4,4'-MDI und/oder 2,4'-MDI ist in polaren Lösungsmitteln gut löslich, es wird weniger in das ausgefällte feste Produkt von Dimeren oder Oligomeren eingebaut.
b) Da das Dimere bei der Reaktionstemperatur gut löslich ist, oligomerisiert das Dimere über die freien Isocyanatgruppen zum oligomeren oder polymeren MDI-Uretdion. Überdies kann sich bei der Synthese und Lagerung das Monomere, Dimere oder Oligomere auch im festen kristallinen Zustand in Produkte mit höherem Molekulargewicht verwandeln, speziell bei Temperaturen im Bereich von 50 - 100 °C.

Die Dimerisierung von Diphenylmethan-4,4'- und/oder 2,4'-diisocyanat ist an sich bekannt, wird aber erfindungsgemäss in besonderen Lösungsmitteln durchgeführt, wodurch eine vorteilhafte, nahezu verlustfreie Herstellung der gewünschten Dispersionen und Suspensionen möglich wird.

Im ersten Verfahrensschritt wird eine Reaktionsmischung aus Diphenylmethan-4,4'- und/oder 2,4'-diisocyanat und einen Dimerisierungs-Katalysator in dem entsprechenden, vorstehend diskutierten mindestens einem aprotischen polaren Lösungsmittel bereitgestellt.

Diphenylmethan-4,4'- und 2,4'-diisocyanat resp. deren Gemische sind kommerziell erhältlich und hinlänglich bekannt. Die vorstehend diskutierten aprotischen polaren Lösungsmittel sind ebenfalls kommerziell erhältlich und hinlänglich bekannt.

Der Dimerisierungs-/Oligomerisierungskatalysator ist ein Katalysator, der die Dimerisierung von Diphenylmethan-4,4'-und/oder 2,4'-diisocyanat zum entsprechenden Uretdion katalysiert. Es kommen hierbei die vorstehend bereits beschriebenen Katalysatoren in Frage.

Die zu wählenden Mengen an Lösungsmittel und Katalysator sind dem Fachmann prinzipiell bekannt und können problemlos den jeweiligen Bedürfnissen entsprechend ausgewählt werden.

Die Dimerisierungs-/Oligomerisierungsreaktion wird im Temperaturbereich von 20 bis 120°C, bevorzugt 20 bis 100 °C, noch mehr bevorzugt zwischen 50 °C und 90 °C, während 1 bis 8 Stunden durchgeführt.

Im Verlauf der Reaktion fällt das oligomere MDI-Uretdion als feines weisses Pulver aus.

Nach Ende der Reaktion wird die entstehende lösungsmittelhaltige Suspension von MDI-Uretdion auf Raumtemperatur gekühlt, und gegebenenfalls der Katalysator zerstört oder neutralisiert.

Anschliessend wird mit einer definierten Menge Wasser verdünnt und das organische Lösungsmittel bei der ersten Ausführungsform weitgehend bis vollständig entfernt, während bei der zweiten Ausführungsform keine Entfernung des organischen Lösungsmittels durchgeführt wird. Es wird also je nach Ausführungsform eine wässrige Suspension/Dispersion oder eine Suspension in Wasser und dem aprotischen polaren Lösungsmittel erhalten.

Der wässrigen Phase werden üblicherweise Additive zugesetzt. Als Beispiele seien genannt:
Entschäumer, wie bspw. Agitan 282 (Münzing Chemie), Dow Corning Antifoam RD Solution (Dow Corning);
Dispergatoren, wie bspw. Tamol NN 4501 (BASF), Dispersogen HR (Clariant);
Emulgatoren, wie bspw. Tanemul FD liq. (Tanatex Chemicals), Genapol X 060 (Clariant), Tergitol TMN6 (Dow Chemical), Lutensit A-BO (BASF);
Aliphatische Amine, wie bspw. Jeffamine T 403 (Huntsman) Diisopropanolamin (BASF), in Konzentrationen von 2 - 10, bevorzugt 3 - 6 Equivalentprozent, bezogen auf den Isocyanatgehalt des MDI;
Rheologische Hilfsmittel, wie bspw. BorchiGel ALA (Borchers), Attagel 40 (BASF). ,

Je nach Anwendung können diesen Dispersionen auch Katalysatoren für die Reversion des Uretdionringes zu niedrigmolekularen Oligomeren, Dimeren oder Monomeren des MDI zugefügt werden. Bevorzugt werden solche Katalysatoren, welche hydrolysenstabil sind. Die Katalysatoren können in einer Menge zwischen 0,001 und 1 Gewichtsprozenten, bezogen auf das MDI-Uretdion, eingesetzt werden.

Katalytisch wirkende tertiäre Amine und metallische Verbindungen sind dem Fachmann bekannt. Beispiele für anorganische oder metallorganische Katalysatoren sind Verbindungen von Calzium, Cobalt, Eisen, Mangan, Wismut, Zink, Zinn, Zirkon.

Tertiäre Amine, welche als Dimerisierungs/Oligomerisierungskatalysatoren für die Uretdionbildung eingesetzt werden, haben sich auch als Reversionskatalysatoren bewährt. Beispiele sind u. a. Amidine, wie Diazabicylcloundecen DBU oder -nonen DBN, 4-Dimethylamino-pyridin, Dimethyl-tetrahydropyrimidin, 1,2-Dimethylimidazol. Siehe auch DE 39 38 203 und US-2006/0247341, deren Offenbarung bezüglicher Reversionskatalysatoren in die vorliegende Beschreibung durch Bezugnahme eingeschlossen wird.

Überraschenderweise hat sich gezeigt, dass auch polymere oder copolymere tertiäre Amine wirksam sein können. Als Vertreter seien hier copolymere Dispersionen von Butadien, Styrol und 2-Vinylpyridin genannt, wie beispielsweise Pyratex 240 oder Pyratex 241 (Polymer Latex).

Weitere Zusätze zu den Dispersionen und ihre Funktionen sind dem Fachmann bekannt.

Falls nötig kann vor oder nach Zugabe von Zusatzstoffen beziehungsweise vor oder nach Entfernen des Lösungsmittels, eine Kornzerkleinerung in einer Mühle (Perlmühle, Sandmühle, Kugelmühle etc.) auf eine mittlere Partikelgrösse von 1 - 10 µm, bevorzugt 2 - 5 µm erfolgen.

Die Dispersion kann während einigen Tagen nach Herstellung kleine Mengen von Kohlendioxid entwickeln. Die CO₂-Entwicklung kann durch Zugabe von Ammoniak oder tertiären Aminen, wie Triethylamin, weitestgehend verhindert werden.

Nach dem erfindungsgemässen Verfahren werden bei der Aufarbeitung praktisch keine Verluste beobachtet.

Die Dispersionen gemäss der ersten Ausführungsform sind lager-und transportfähig, bei vierwöchiger Lagerung bei 40 °C findet kein Rückgang des Gesamtgehaltes von Isocyanatgruppen statt.

Die 30 bis 60 %-ige Suspension von MDI-Uretdion in Wasser und Lösungsmittel gemäss der zweiten Ausführungsform ist ebenfalls lagerstabil und wird in dieser Form in den Handel gebracht.

Die Dispersionen werden unmittelbar vor Gebrauch als Vernetzungs- oder Haftmittel mit der 10 - bis 100-fachen Menge Wasser versetzt.

Für die Verwendung als Haftvermittler für Verstärkungsfasern für Reifen, Transportbänder oder Antriebsriemen werden weitere reaktive Komponenten zugegeben, wie zum Beispiel Epoxydverbindungen, Resorcin-Formaldehyd Lösungen, und Styrol-Butadien-Vinylpyridin-Copolmyer Latex. Das polare Lösungsmittel bildet bei der zweiten erfindungsgemässen Ausführungsform mit dem Wasser vorzugsweise ein Azeotrop, welches sich bei der Trocknung bei Raumtemperatur oder bei erhöhten Temperaturen im Bereich 30 °C bis 250 °C verflüchtigt. Durch den Wasserüberschuss zeigen solche Mischungen auch keinen Flammpunkt unter 65 °C.

Um den Isocyanatgehalt zu bestimmen, für die DSC- oder Infrarotanalyse, können die MDI-Uretdione als Pulver aus der Suspension durch Filtrieren oder Zentrifugieren und nachfolgendes Waschen und Trocknen gewonnen werden. An der Suspension, gegebenenfalls verdünnt mit Wasser, lässt sich leicht die Partikelgrössenverteilung bestimmen.

Die Bestimmung des Gesamtgehalts an NCO-Gruppen am trockenen MDI-Uretdion wird mit der Umsetzung mit einem Überschuss an Dibutylamin durchgeführt. Dibutylamin ist im Dimethylformamid gelöst und wird 3 Stunden auf Siedetemperatur des DMF gehalten. Es wird nach bekannten Verfahren ein Gehalt von 24 bis 30 % NCO bestimmt, bei einem theoretischen Gehalt von 33 %. Die Abweichung wird dadurch bestimmt, dass die endständigen Isocyanatgruppen der Oligomere mit Wasser reagieren können. Je nach Katalysator bilden sich auch Nebenprodukte, zum Beispiel Trimere (Isocyanurate), die einen viel niedrigeren Isocyanatgehalt aufweisen.

Der gefundene hohe Gehalt an Isocyanatgruppen nach Rückspaltung der Uretdiongruppen bei der NCO-Analyse weist darauf hin, dass mit dem erfindungsgemässen Verfahren aus 4,4'-MDI oder aus Gemischen von 4,4'-MDI mit 2,4'-MDI überwiegend MDI-Uretdion-Oligomere mit Polymerisationsgrad > 3 hergestellt werden. Freie endständige Isocyanatgruppen, welche nicht in das feste Uretdion eingebunden sind, reagieren mit dem Wasser unter Harnstoffbildung, respektive mit dem aliphatischen, primären oder sekundären Amin, welches der wässerigen Phase zugesetzt wird.

Nach Trocknung und Vernetzung in der Wärme dienen die erfindungsgemässen Dispersionen von MDI-Uretdionen als Haftvermittler und Vernetzungsmittel für gelöste, dispergierte und feste Polymere oder Elastomere, gegebenenfalls mit funktionellen Gruppen, bei Temperaturen von Normaltemperatur bis 300 °C, wobei zusätzliche nieder- oder hochmolekulare Verbindungen mit funktionellen Gruppen mitreagieren können.

Die vorliegende Erfindung betrifft somit die Verwendung einer wie vorstehend beschrieben erhältlichen fliessfähigen wässrigen Suspension oder Dispersion von oligomeren Diphenylmethan-4,4'- und/oder -2,4'-diisocyanat-uretdionen, welche keinen Flammpunkt mehr aufweist, als Haftvermittler zur Verbesserung der Haftfestigkeit zwischen einer textilen Verstärkungseinlage und einem Kautschuk in einem faserverstärkten Kautschukprodukt.

Die vorliegende Erfindung betrifft ebenfalls die Verwendung einer wie vorstehend beschrieben erhältlichen Suspension aus mindestens einem polaren aprotischen Lösungsmittel und Wasser, wobei das polare aprotische Lösungsmittel wasserlöslich oder wassermischbar ist und unter Normalbedingungen einen Siedepunkt von 100°C bis 250°C aufweist, als Haftvermittler zur Verbesserung der Haftfestigkeit zwischen einer textilen Verstärkungseinlage und einem Kautschuk in einem faserverstärkten Kautschukprodukt.

Erfindungsgemäss werden die Dispersionen insbesondere verwendet zur Haftvermittlung zwischen Reifencord und Kautschuk.

Die vorliegende Erfindung wird nachstehend anhand von nicht einschränkenden Beispielen näher erläutert.

### Beispiel 1:

### Herstellung einer 30 %-igen 4,4'-MDI-Uretdion Dispersion in Wasser

### 1.1. MDI-Uretdionsuspension in Aceton

In einem 0.5 L Reaktionsgefäss mit Ankerrührer wurden unter einem leichten Stickstoffstrom 155 g 4,4'-Diphenylmethandiisocyanat (= 1.24 Equivalente) in 150 g Aceton (mit 0.1 % Wasser im Aceton) bei Raumtemperatur gelöst und die Temperatur auf 56 °C erhöht. Nach 1 Stunde Entwässerungszeit wurde 0.35 g Tributylphosphin zugegeben und bei 56 °C (Siedetemperatur des Acetons) während 6.5 Stunden zur Reaktion gebracht. Das MDI-Uretdion schied sich als suspendiertes feines weisses Pulver mit einer mittleren Partikelgrösse von 12 µm ab. Es bildete sich eine fliessfähige thixotrope Suspension.

Nach Ende der Reaktionszeit wurde der Ansatz bei Raumtemperatur über Nacht gerührt und dann 1 Stunde auf eine Temperatur von 15°C gekühlt. Anschliessend wurden 0.35 g p-Toluolsulfonsäuremethylester zugegeben, um den Phosphinkatalysator zu zerstören. Der Ansatz wurde während 15 Minuten weiter gerührt. Ein leichter Verlust von Aceton wurde wieder ergänzt.

Das totale Gewicht der Suspension in Aceton betrug 305.7 g mit ungefähr 51 % Feststoff.

### 1.2. Herstellen der wässrigen Phase

### a) Wässrige Stammlösung

Es wurden 60.0 g einer Stammlösung zubereitet, enthaltend

| | |
|---|---|
| Diisopropanolamin (BASF) | 6.40 g (0.0481 Amin-eq.) |
| Tamol NN 4501 (BASF) | 9.00 g |
| Antifoam RD Emulsion (Dow Corning) | 0.38 g |
| Wasser | 44.22 g |

### b) Wässrige Phase

60 g der Stammlösung aus Bsp. 1.2.a) wurden mit 285 g Wasser verdünnt, so dass eine totale wässrige Phase von 345 g erhalten wurde.

### 1.3. Herstellung der Dispersion von MDI-Uretdion in Wasser / Aceton

345 g wässrige Phase aus Bsp. 1.2 wurden in einem 1 L Becherglas vorgelegt und mit einem Dissolverrührer gerührt, welcher mit 750 Umdrehungen pro Minute rotierte. In die vorgelegte wässrige Phase wurden 305.7 g der MDI-Uretdion Suspension in Aceton aus Bsp. 1.1. einemulgiert, welche 650.7 g einer fliessfähigen, leicht thixotropen Dispersion (10) ergaben. Die Dispersion wurde noch 15 Minuten bei 1000 Umdrehungen pro Minute weitergerührt.

Mit der angegeben Menge von Diisopropanolamin konnten 3.9 Equivalent-% NCO-Gruppen der MDI-Einwaage oberflächendesaktiviert werden.

### 1.4. Aufbereiten der Dispersion

### a) Mahlen der Dispersion

Die Dispersion wurde unter Mitverwendung eines gleichen Volumenteils Glaskugeln von 2 mm Durchmesser in einer Perlmühle, welche mit 1800 Umdrehungen pro Minute rotierte, auf 3.5 µm mittlere Partikelgrösse reduziert.

### b) Abdestillieren von Aceton

Das Aceton wurde aus der MDI-Uretdiondispersion bei 48 °C und einem Druck von 170 mbar unter Verwendung eines Rotationsverdampfers abdestilliert, wobei eine ungefähr 30 %-ige Dispersion entstand. Der Restgehalt des Acetons in der Dispersion betrug 2.4 %, wodurch das Produkt keinen Flammpunkt zeigte. Die Dispersion entwickelte wenig CO₂, nach etwa einer Woche hörte die CO₂-Entwicklung auf.

### c) Pro 100 g Dispersion wurden anschliessend 1 % BorchiGel ALA eingerührt, wobei eine fliessfähige, thixotrope Dispersion resultierte.

Am trockenen Pulver wurde ein totaler NCO-Gehalt von 26 % gemessen. Die Bestimmung wurde mit der Dibutylamin-Methode in Dimethylformamid vorgenommen, wobei die Lösung (vor dem Rücktitrieren des Dibutylamins mit 0.1 n HCl) während 3 Stunden bei Siedetemperatur des DMF gehalten wurde.

Bei Lagerung bei 40 °C während 28 Tagen konnte kein Abfall des NCO-Gehaltes festgestellt werden.

Aufgrund von ausgedehnten Versuchen wurde gezeigt, dass diese erfindungsgemässe circa 30 %-ige wässrige Dispersion von MDI-Uretdion anstelle einer 50 %-igen Dispersion von Caprolactam-blockierten MDI als Haftvermittler zur Verbesserung der Haftfestigkeit zwischen textiler Verstärkungseinlage und dem Kautschuk verwendet werden kann. Sie eignet sich auch als heisshärtende Vernetzungskomponente für wässrige Dispersionen von Polymeren, welche isocyanatreaktive Gruppen enthalten.

### Beispiel 2:

### Herstellung einer 30 % 4,4'-MDI-Uretdionsuspension in Dipropylenglykoldimethlyether und Wasser

### 2.1. MDI-Uretdionsuspension in Dipropylenglykoldimethlyether

Die Synthese wurde in einem 0.5 ml Glasreaktor mit Ankerrührer durchgeführt, ausgerüstet mit wassergekühltem Kugelkühler, thermostatiert mit heizbarem Wasserbad, unter leichtem Stickstoffstrom.

200 g Dipropylenglykoldimethylether wurden vorgelegt und 255 g (2.04 Equivalente) 4,4'-MDI Schuppen bei Raumtemperatur zugegeben, unter Rühren auf 65 °C erwärmt und zur Entwässerung des Lösungsmittels 1 Stunde bei 65 °C gehalten.

Mit einer Spritze wurden anschliessend 0.50 g Tributylphosphin zugegeben, so dass sich eine Mischung mit einem Gesamtgewicht von 455.5 g ergab. Die Temperatur wurde während 6.5 Stunden bei 75°C gehalten. Das MDI-Uretdion schied sich als suspendiertes feines weisses Pulver mit einer mittleren Partikelgrösse von 15 µm ab. Es bildete sich eine fliessfähige thixotrope Suspension.

Nach Ende der Reaktionszeit wurde der Ansatz bei Raumtemperatur über Nacht gerührt und dann 1 Stunde auf eine Temperatur von 15°C gekühlt. Anschliessend wurden 0.50 g p-Toluolsulfonsäuremethylester zugegeben, um den Phosphinkatalysator zu zerstören. Die Mischung wurde 15 Minuten bei Raumtemperatur rühren lassen. In der Gesamtmenge von etwa 456.0 g waren etwa 56.1 % oligomeres 4,4'-MDI-Uretdion und 43.8 % Lösungsmittel DMM enthalten.

### 2.2. Herstellung einer Dispersion von MDI-Uretdion in Wasser / Dipropylenglykoldimethlyether

### a) Wässrige Stammlösung

Es wurden 100.000 g einer Stammlösung zubereitet, enthaltend 10.625 g (0.08 Equivalente) Diisopropanolamin (BASF), 15.000 g Tamol NN 4501 (BASF), 0.625 g Antifoam RD Emulsion (Dow Corning), 6.250 g Tergitol TMN-6 und 67.500 g Wasser.

### b) Wässrige Phase

100.0 g der Stammlösung aus Bsp. 2.2.a) wurden mit 276.5 g Wasser verdünnt, so dass eine totale Wässrige Phase von 376.5 g erhalten wurde.

### c) Herstellung der MDI-Uretdion Dispersion in Wasser / Dipropylenglykoldimethlyether

376.5 g der wässrigen Phase aus Bsp. 2.2.b) wurde in einem 1.5 L Becherglas vorgelegt und mit einem Dissolverrührer gerührt, welcher mit 750 Umdrehungen pro Minute rotierte. In die vorgelegte wässrige Phase wurden 456.0 g der MDI-Uretdion Suspension DMM aus Bsp. 2.1. einemulgiert, welche 832.5 g einer fliessfähigen, leicht thixotropen Dispersion ergaben.

Die Dispersion wurde noch 15 Minuten bei 1000 Umdrehungen pro Minute weiter gerührt.

Mit der angegeben Menge von Diisopropanolamin konnten 3.9 Equivalent-% NCO-Gruppen der MDI-Einwaage oberflächendesaktiviert werden.

Endprodukt war eine Dispersion mit ungefähr 30.7 % Feststoff MDI-Uretdion und 24 % Dipropylenglykoldimethylether und 45.2 % Wasser.

### 2.3. Aufbereiten der Dispersion

Die Dispersion wurde unter Mitverwendung eines gleichen Volumenteils Glaskugeln von 2 mm Durchmesser in einer batchweise arbeitenden Perlmühle, welche mit 1800 Umdrehungen pro Minute rotierte, auf 4.9 µm mittlere Partikelgrösse reduziert. Die Mahlzeit betrug 10 Minuten.

Pro 100 g Dispersion wurden anschliessend 0.5 g Attagel 40 eingerührt, wobei eine thixotrope pseudoplastische Dispersion resultierte.

Die Dispersion entwickelte wenig CO₂, nach etwa einer Woche hörte die CO₂-Entwicklung auf.

Am trockenen Pulver wurde ein totaler NCO-Gehalt von 27.7 % gemessen. Die Bestimmung wurde mit der Dibutylamin-Methode in Dimethylformamid vorgenommen, wobei die Lösung (vor dem Rücktitrieren des Dibutylamins mit 0.1 n HCl) während 3 Stunden bei Siedetemperatur des DMF gehalten wurde.

Bei Lagerung bei 40 °C während 28 Tagen konnte kein Abfall des NCO-Gehaltes festgestellt werden.

Aufgrund von ausgedehnten Versuchen wurde gezeigt, dass diese erfindungsgemässe circa 30 %-ige Dispersion von MDI-Uretdion in Wasser / Dipropylenglykoldimethylether anstelle einer 50 %-igen Dispersion von Caprolactam-blockierten MDI als Haftvermittler zur Verbesserung der Haftfestigkeit zwischen textiler Verstärkungseinlage und dem Kautschuk verwendet werden kann.

Sie eignet sich auch als heisshärtende Vernetzungskomponente für wässerige Dispersionen von Polymeren, welche isocyanatreaktive Gruppen enthalten.

## Patentansprüche

1. Verfahren zur Herstellung einer Dispersion oder Suspension von oligomeren Diphenylmethan-4,4'-diisocyanat-uretdionen und/oder Diphenylmethan-2,4'-diisocyanat-uretdionen, umfassend die aufeinander folgenden Schritte
a) Bereitstellung einer Reaktionsmischung enthaltend Diphenylmethan-4,4'-diisocyanat und/oder Diphenylmethan-2,4'-diisocyanat; und einen Dimerisierungs-Katalysator in einem aprotischen polaren Lösungsmittel oder einem Gemisch aprotischer polarer Lösungsmittel;
b) Durchführung der Dimerisierungs-/Oligomerisierungsreaktion ;
c) gegebenenfalls Neutralisation oder Zerstörung des Katalysators;
**dadurch gekennzeichnet, dass**
i) das polare aprotische Lösungsmittel wasserlöslich oder wassermischbar ist und unter Normalbedingungen einen Siedepunkt von 40°C bis 100°C aufweist oder mit Wasser ein unter 100°C siedendes Azeotrop bildet, und das Lösungsmittel nach Reaktionsende zumindest so weit durch Wasser ersetzt wird, dass eine fliessfähige wässrige Suspension oder Dispersion erhalten wird, die keinen Flammpunkt mehr aufweist;
oder
ii) das polare aprotische Lösungsmittel wasserlöslich oder wassermischbar ist und unter Normalbedingungen einen Siedepunkt von 100°C bis 250°C aufweist.

2. Verfahren nach Anspruch 1,
**dadurch gekennzeichnet, dass** bei der Alternative ii) nach Abschluss der Dimerisierungs-/Oligomerisierungsreaktion Wasser oder eine Lösung eines primären oder sekundären Amins oder Polyamins in Wasser zugegeben wird, um freie Isocyanatgruppen zu entfernen.

3. Verfahren nach Anspruch 1 oder 2,
**dadurch gekennzeichnet, dass** das Versetzen mit Wasser und/oder eine Lösung eines primären oder sekundären Amins oder Polyamins in Wasser nach Reaktionsabschluss **dadurch** erfolgt, dass die erhaltene Mischung in der vorgelegten wässrigen Phase dispergiert wird.

4. Verfahren nach einem der Ansprüche 1 bis 3,
**dadurch gekennzeichnet, dass** die erhaltenden Uretdion-Partikel auf eine Korngrösse von weniger als 10 µm zerkleinert werden.

5. Verfahren nach einem der Ansprüche 1 bis 4,
**dadurch gekennzeichnet, dass** der Dimerisierungs-/Oligomerisierungskatalysator ausgewählt ist aus der Gruppe bestehend aus Trialkylphosphinen, Tris-(dialkyamino)-phosphinen, substituierten Pyridinen, substituierten Imidazolen, tertiären Aminen, heterocyclischen Aminen und Amidinen.

6. Verfahren nach einem der Ansprüche 1 bis 5,
**dadurch gekennzeichnet, dass** der erhaltenen Dispersion oder Suspension Zusatzstoffe zugesetzt werden.

7. Verfahren nach Anspruch 6,
**dadurch gekennzeichnet, dass** es sich bei den Zusatzstoffen um Katalysatoren für die Reversion des Uretdionringes zu niedrigmolekularen Oligomeren, Dimeren oder Monomeren des Diphenylmethan-4,4'-diisocyanat und/oder Diphenylmethan-2,4'-diisocyanat handelt.

8. Verfahren nach einem der Ansprüche 1 bis 7,
**dadurch gekennzeichnet, dass** bei der Ausführungsform i) das Lösungsmittel durch Abdestillieren bei Normaldruck oder unter vermindertem Druck ; Versprühen des Gemisches unter vermindertem Druck mit weitgehender oder vollständiger Entfernung des Lösungsmittels; oder durch ein- oder mehrmaliges Zentrifugieren, Verwerfen der flüssigen Phase und Aufschlämmen des MDI-Uretdionpulvers mit Wasser weitgehend oder vollständig entfernt wird.

9. Verfahren nach einem der Ansprüche 1 bis 8,
**dadurch gekennzeichnet, dass** bei der Ausführungsform i) das Lösungsmittel ausgewählt ist aus der Gruppe bestehend aus Aceton, Methylethylketon, Tetrahydrofuran, 2-Methyltetrahydrofuran, 1,2-Dimethoxyethan, Dioxan und Methylacetat.

10. Verfahren nach einem der Ansprüche 1 bis 9,
**dadurch gekennzeichnet, dass** bei der Ausführungsform ii) das Lösungsmittel ausgewählt ist aus der Gruppe bestehend aus Diethylenglykol-diethylether, Diethylenglykol-dimethylether, Propylenglykoldimethylether, Dipropylenglykol-dimethylether, N-Methyl-pyrrolidon, N-Ethyl-2-pyrrolidon, Cyclohexanon und Ethylen-/Propylencarbonat (50 % Gemisch).

11. Verfahren gemäss einem der Ansprüche 1 bis 10,
**dadurch gekennzeichnet, dass** die Suspension oder Dispersion auf einen Wassergehalt von > 50 Gew.%.

12. Verfahren zur Verbesserung der Haftfestigkeit zwischen einer textilen Verstärkungseinsge und einem Kautsschuk it einem faserverstärkler Kantschakprodukt, umfassend die Schritte :
- Herstellen einer fliessfähigen Suspension oder Dispersion von oligomeren Diphenylmethan-4,4'- und/oder Diphenylmethan-2,4'-diisocyanat-uretdionen, welche keinen Flammpunkt mehr aufweist mit einem Verfahren gemäss einem der Ansprüche 1 bis 10, oder einer Suspension enthaltend mindestens ein polares aprotisches Lösungsmittel und Wasser, wobei das polare aprotische Lösungsmittel wasserlöslich oder wassermischbar ist und unter Normalbedingungen einen Siedepunkt von 100°C bis 250°C aufweist mit einem Verfahren gemäss einem der Ansprüche 1 bis 11;
- Verwendung der Suspension oder Dispersion als Haftvermittler zwischen die textile Verstärkungseinlage und der Kautschuk.

13. Verfahren gemäss Anspruch 12,
**dadurch gekennzeichnet, dass** die Suspension oder Dispersion > 50 Gew.% Wasser enthält.

14. Verfahren nach einem der Ansprüche 12 bis 13,
**dadurch gekennzeichnet, dass** es sich bei der textilen Verstärkungslage um Reifencord und bei dem faserverstärkten Kautschukprodukt um einen Reifen handelt.

## Claims

1. Method for producing a dispersion or suspension of oligomeric diphenylmethane 4,4'-diisocyanate uretdiones and/or diphenylmethane 2,4'-diisocyanate uretdiones, comprising the consecutive steps of
a) providing a reaction mixture comprising diphenylmethane 4,4'-diisocyanate and/or diphenylmethane 2,4'-diisocyanate; and a dimerization catalyst in an aprotic polar solvent or a mixture of aprotic polar solvents;
b) conducting the dimerization/oligomerization reaction;
c) optionally neutralizing or destroying the catalyst;
**characterized in that**
i) the polar aprotic solvent is water-soluble or water-miscible and has a boiling point of 40°C to 100°C under standard conditions or constitutes with water an azeotrope boiling below 100°C, and the solvent is replaced, after the reaction has ended, with at least sufficient water to obtain a flowable aqueous suspension or dispersion that does not have a flash point;
or
ii) the polar aprotic solvent is water-soluble or water-miscible and has a boiling point of 100°C to 250°C under standard conditions.

2. Method according to Claim 1,
**characterized in that**, in alternative ii), after conclusion of the dimerization/oligomerization reaction, water or a solution of a primary or secondary amine or polyamine in water is added to remove free isocyanate groups.

3. Method according to Claim 1 or 2, **characterized in that** the admixing with water and/or a solution of a primary or secondary amine or polyamine in water after conclusion of the reaction is effected by dispersing the resulting mixture in the provided aqueous phase.

4. Method according to any one of Claims 1 to 3, **characterized in that** the uretdione particles obtained are comminuted to a particle size of less than 10 µm.

5. Method according to any one of Claims 1 to 4, **characterized in that** the dimerization/- oligomerization catalyst is selected from the group consisting of trialkylphosphines, tris(dialkylamino)phosphines, substituted pyridines, substituted imidazoles, tertiary amines, heterocyclic amines and amidines.

6. Method according to any one of Claims 1 to 5, **characterized in that** additional substances are added to the dispersion or suspension obtained.

7. Method according to Claim 6,
**characterized in that** the additional substances comprise catalysts for reversion of the uretdione ring to low molecular weight oligomers, dimers or monomers of diphenylmethane 4,4'-diisocyanate and/or diphenylmethane 2,4'-diisocyanate.

8. Method according to any one of Claims 1 to 7, **characterized in that**, in embodiment i), the solvent is substantially or completely removed by distillative removal at standard pressure or under reduced pressure; spray-dispensing the mixture under reduced pressure with substantial or complete removal of the solvent; or by single or multiple centrifuging, discarding of the liquid phase and slurrying of the MDI-uretdione powder with water.

9. Method according to any one of Claims 1 to 8, **characterized in that**, in embodiment i), the solvent is selected from the group consisting of acetone, methyl ethyl ketone, tetrahydrofuran, 2-methyltetrahydrofuran, 1,2-dimethoxyethane, dioxane and methyl acetate.

10. Method according to any one of Claims 1 to 9, **characterized in that**, in embodiment ii), the solvent is selected from the group consisting of diethylene glycol diethyl ether, diethylene glycol dimethyl ether, propylene glyol dimethyl ether, dipropylene glycol dimethyl ether, N-methylpyrrolidone, N-ethyl-2-pyrrolidone, cyclohexanone and ethylene/propylene carbonate (50% mixture).

11. Method according to any one of Claims 1 to 10, **characterized in that** the suspension or dispersion is adjusted to a water content of > 50% by weight.

12. Method for improving the adherence between a textile reinforcing ply and a rubber in a fibre-reinforced rubber product, comprising the steps of:
- producing a flowable suspension or dispersion of oligomeric diphenylmethane 4,4'- and/or diphenylmethane 2,4'-diisocyanate uretdiones which does not have a flash point using a method according to any one of Claims 1 to 10 or a suspension containing at least one polar aprotic solvent and water, wherein the polar aprotic solvent is water-soluble or water-miscible and has a boiling point of 100°C to 250°C under standard conditions using a method according to any one of Claims 1 to 11;
- using the suspension or dispersion as an adhesion promoter between the textile reinforcing ply and the rubber.

13. Method according to Claim 12,
**characterized in that** the suspension or dispersion contains > 50% by weight of water.

14. Method according to either of Claims 12 to 13, **characterized in that** the textile reinforcing ply is a tyre cord and the fibre-reinforced rubber product is a tyre.

## Revendications

1. Procédé de préparation d'une dispersion ou d'une suspension de diphénylméthane-4,4'-diisocyanatouretdiones et/ou de diphénylméthane-2,4'-diisocyanatouretdiones oligomères, comprenant les étapes consécutives suivantes
a) mise à disposition d'un mélange réactionnel contenant du diphénylméthane-4,4'-diisocyanate et/ou du diphénylméthane-2,4'-diisocyanate ; et d'un catalyseur de dimérisation dans un solvant polaire aprotique ou un mélange de solvants polaires aprotiques ;
b) réalisation de la réaction de dimérisation/oligomérisation ;
c) le cas échéant, neutralisation ou décomposition du catalyseur ;
**caractérisé en ce que**
i) le solvant polaire aprotique est soluble dans l'eau ou miscible à l'eau et présente, dans les conditions normales, un point d'ébullition de 40°C à 100°C ou forme avec l'eau un azéotrope bouillant au-dessous de 100°C, et le solvant est remplacé après la fin de la réaction par de l'eau au moins dans une mesure telle qu'on obtienne une suspension ou une dispersion aqueuse pouvant s'écouler, qui ne présente plus de point d'éclair ; ou
ii) le solvant polaire aprotique est soluble dans l'eau ou miscible à l'eau et présente, dans les conditions normales, un point d'ébullition de 100°C à 250°C.

2. Procédé selon la revendication 1, **caractérisé en ce que** dans le cas de l'alternative ii), après la fin de la réaction de dimérisation/oligomérisation, on ajoute de l'eau ou une solution d'une amine ou d'une polyamine primaire ou secondaire dans l'eau, pour éliminer les groupes isocyanate libres.

3. Procédé selon la revendication 1 ou 2, **caractérisé en ce que** l'addition d'eau et/ou d'une solution d'une amine ou d'une polyamine primaire ou secondaire dans l'eau après la fin de la réaction est réalisée **en ce que** le mélange obtenu est dispersé dans une phase aqueuse disposée au préalable.

4. Procédé selon l'une quelconque des revendications 1 à 3, **caractérisé en ce que** les particules d'uretdione obtenues sont broyées à une grosseur des grains inférieure à 10 µm.

5. Procédé selon l'une quelconque des revendications 1 à 4, **caractérisé en ce que** le catalyseur de dimérisation/oligomérisation est choisi dans le groupe constitué par les trialkylphosphines, les tris-(dialkylamino)-phosphines, les pyridines substituées, les imidazoles substitués, les amines tertiaires, les amines et les amidines hétérocycliques.

6. Procédé selon l'une quelconque des revendications 1 à 5, **caractérisé en ce que** des additifs sont ajoutés à la dispersion ou à la suspension obtenue.

7. Procédé selon la revendication 6, **caractérisé en ce qu'**il s'agit, pour les additifs, de catalyseurs pour l'inversion du cycle uretdione en oligomères, dimères ou monomères à bas poids moléculaire du diphénylméthane-4,4'-diisocyanate et/ou du diphénylméthane-2,4'-diisocyanate.

8. Procédé selon l'une quelconque des revendications 1 à 7, **caractérisé en ce que** dans la forme de réalisation i), le solvant est éliminé dans une large mesure ou totalement par distillation à pression normale ou réduite ; par pulvérisation du mélange sous pression réduite avec une élimination importante ou totale du solvant ; ou par centrifugation unique ou multiple, rejet de la phase liquide et mise en suspension de la poudre de MDI-uretdione avec de l'eau.

9. Procédé selon l'une quelconque des revendications 1 à 8, **caractérisé en ce que** dans la forme de réalisation i), le solvant est choisi dans le groupe constitué par l'acétone, la méthyléthylcétone, le tétrahydrofuranne, le 2-méthyltétrahydrofuranne, le 1,2-diméthoxyéthane, le dioxane et l'acétate de méthyle.

10. Procédé selon l'une quelconque des revendications 1 à 9, **caractérisé en ce que** dans la forme de réalisation ii), le solvant est choisi dans le groupe constitué par le diéthylèneglycoldiéthyléther, le diéthylèneglycoldiméthyléther, le propylèneglycoldiméthyléther, le dipropylèneglycoldiméthyléther, la N-méthylpyrrolidone, la N-éthyl-2-pyrrolidone, la cyclohexanone et l'éthylènecarbonate/propylènecarbonate (mélange à 50%).

11. Procédé selon l'une quelconque des revendications 1 à 10, **caractérisé en ce que** la suspension ou la dispersion est réglée à une teneur en eau > 50% en poids.

12. Procédé pour améliorer l'adhésivité entre une pièce intercalaire de renforcement textile et un caoutchouc dans un produit à base de caoutchouc renforcé par des fibres, comprenant les étapes de :
- préparation d'une suspension ou d'une dispersion pouvant s'écouler de diphénylméthane-4,4'-diisocyanato-uretdiones et/ou de diphénylméthane-2,4'-diisocyanato-uretdiones oligomères, qui ne présente plus de point d'éclair, par un procédé selon l'une quelconque des revendications 1 à 10, ou d'une suspension contenant au moins un solvant aprotique polaire et de l'eau, où le solvant aprotique polaire est soluble dans l'eau ou miscible à l'eau et présente dans les conditions normales un point d'ébullition de 100°C à 250°C, par un procédé selon l'une quelconque des revendications 1 à 11 ;
- utilisation de la suspension ou de la dispersion comme promoteur d'adhérence entre la pièce intercalaire de renforcement textile et le caoutchouc.

13. Procédé selon la revendication 12, **caractérisé en ce que** la suspension ou la dispersion contient > 50% en poids d'eau.

14. Procédé selon l'une quelconque des revendications 12 a' 13, **caractérisé en ce qu'**il s'agit, pour les pièces intercalaires de renforcement textiles, de tissus pour pneumatiques ou, pour le produit à base de caoutchouc renforcé par des fibres, de pneumatiques.
